# EUROPEAN PATENT APPLICATION

(11) **EP 1 174 147 A1**
(43) Date of publication of application: **23.01.2002**
(21) Application number: 00113551.6
(22) Date of filing: 27.06.2000
(51) Int. Cl.: A61K 38/47, A61P 31/04

(54) **Reversal of antibiotic resistance with lysozyme dimer**

(71) Applicant: NIKA HEALTH PRODUCTS LIMITED, FL-9490 Vaduz (LI)
(72) Inventor: Kiczka, Witold, Prof., Pennington, NJ 08534 (US)
(74) Representative: Büchel, Kurt F., Dr.

(57) **Abstract**

The present invention relates to the use of lysozyme dimer for rendering antibiotic-resistant microbes antibiotic-sensitive and to the use of lysozyme dimer as an active ingredient in the manufacture of a pharmaceutical composition for adminstration to a recipient suffering from an antibiotic-resistant microbial infection for restoring the recipient's susceptibility towards antibiotic treatment, wherein the microbes are resistant to one or more antibiotics and wherein susceptibility toward at least one antibiotic is restored.

## Description

### TECHNICAL FIELD

The invention relates to a method for rendering antibiotics-resistant microbes again susceptible to antibiotics treatment. It further relates to a method of treating human or animal individuals suffering from an antibiotics-resistant microbial infection and to the use of lysozyme dimer in the manufacture of a medicament for such treatment.

### BACKROUND OF THE INVENTION:

Lysozyme dimer is known to be prophylactically and therapeutically effective as an antiviral, antibacterial, immunostimulating and immunomodulating agent. Its benefical properties, utilities and a method of manufacture for the purified substance have been disclosed in WO 89/11294, WO 91/10731, WO 94/01127 and WO 96/21463, respectively.

For various reasons including wide-spread abuse of conventional antibiotics both by patients and medical practitioners and non-compliance of patients with prescribed drug administration schemes, contemporary medicine faces considerable difficulties in providing effective medical treatment for patients and medical personal afflicted with acute or chronical microbial infections by antibiotics-resistant strains. Indeed, very often pathogenic strains have become multi-resistant against a number of antibiotics and some strains have even acquired total resistance against all currently available antibiotics. As a consequence, an increasing number of infectious diseases has turned to a serious problem, particularly to patients and staff in hospitals worldwide.

One of the most common causes of hospital infections is the gram-positive bacterium *Staphylococcus aureus.* Infections with methicillin-resistant *S*. *aureus* (MRSA) strains were first described already in 1961 (1). MRSA strains are multiresistant, i.e. they are resistant to all β-lactamase antibiotics as well as to aminoglycosides, macrolides, lincosamides, and tetracyclines. Methicillin resistance in staphylococci is due to the acquisition of the chromosomal gene mecA and its flanking sequences (mecDNA) (2). In addition, these strains are frequently resistant to chemotherapeutics such as co-trimoxazole and quinolons (3). Consequently, treatment of infections caused by MRSA is extremely difficult because the only antibiotics that can be used are glycopeptides, i.e. vancomycin and teicoplanine. However, strains that are only moderately responsive to them (glycopeptide intermediate *S.aureus,* or GISA) have been identified in Japan and the USA, which may pose significant problems in the treatment of infections caused by these bacteria (4).

MSSA (methicillin susceptible *S.aureus)* and MRSA strains are considered to be potentially epidemic, particularly in hospitals. They have been proven to spread not only within individual hospital departments, but also between hospital departments and even between hospitals. Furthermore, some of the MRSA clones were identified as causing epidemics within entire countries or continents (5). Since 1980, a steady increase in hospital epidemics caused by MRSA has been observed. In Poland, MRSAs can be found and isolated in each hospital. The percentage of MRSA isolates exceeds 30% in Italy, France and Spain, and the data collected in the USA place the rate at approximately 19% (6).

Thus tremendous efforts are undertaken in the scientific world and among public health agencies to restrain antibiotic resistances, since novel antibiotics are few and unfortunately induce new resistances after some time.

### SUMMARY OF THE INVENTION

The present invention comprises methods and compositions for recovering the susceptibility of antibiotics-resistant microbes toward at least one of the antibiotics against which the microbes are resistant. The methods comprise administration of lysozyme dimer before, simultaneously with or after the administration of another dose of one or more of the otherwise inefficacious antibiotics. This combinatorial application of lysozyme dimer and antibiotic(s) in cases of antibiotics-resistant microbial infections may cause a partial or full reversal of antibiotics-resistance of the microbes and thus provides for a method to combat hospitalism and to relieve patients from otherwise untreatable or hardly treatable infections.

The benefit of the present invention is exemplified herein with the clinically most relevant, multiple-resistant strains of *S.aureus,* which shall not be understood, however, to limit the invention in any respect.

The invention further comprises the use of lysozyme dimer for the manufacture of a pharmaceutical composition for combined application with one or more antibiotics before, simultaneous with or after administration of the antibiotic(s). The lysozyme dimer may be prepared in any suitable galenic form for oral or parenteral application using a pharmaceutically acceptable carrier and optionally further additives common in the art.

### BRIEF DESCRIPTION OF THE DRAWINGS

Figure 1 shows an electrophoresis of a PCR-amplified portion of the mecA DNA measuring 533 bp (base pairs) in 3% agar gel. The following assays were used: M = DNA size marker; path1 = MRSA strain, MR3 model; paths 2 to 14 = MRSA strains in which the amplification product measuring 533 bp is visible, which corresponds to the size of the mecA gene.
Figure 2 shows the result of a disk-diffusion assay in agar plate cultures of a MSSA strain in MHA medium:
   Plate A, without lysozyme dimer addition to the medium, exhibits no growth inhibition areas around GM (gentamycin), CC (clindamycin) and SXT (co-trimoxazole) disks;
   Plate B, with lysozyme dimer addition (10 µg/ml) to the medium, exhibits clearly visible growth inhibition areas around GM, CC, and SXT disks.

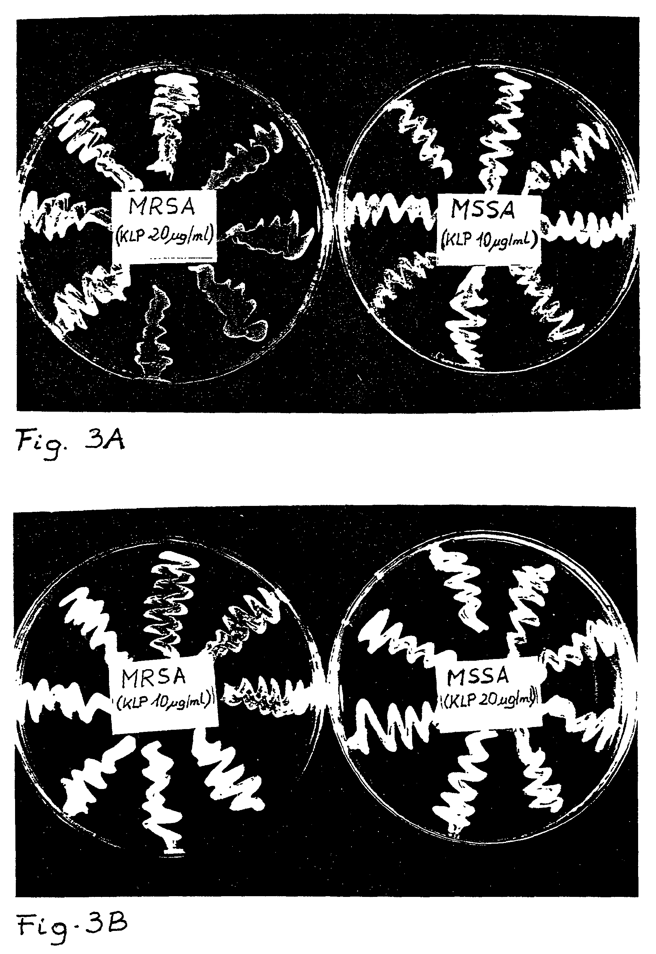

Figures 3A and 3B show control tests with growth of MRSA and MSSA strains in MHA medium in the presence of 10 µg/ml and 20 µg/ml lysozyme dimer (termed "KLP") but in the absence of antibiotics.

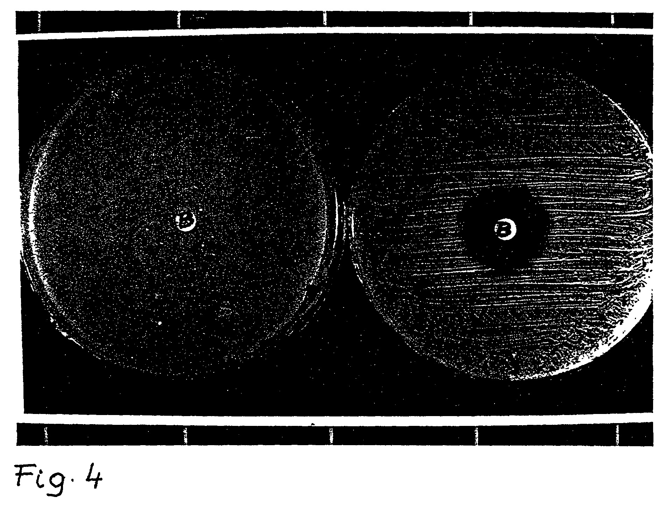

Figure 4 demonstrates the recovery of Biseptol susceptibility of a MRSA strain after 18 hours of incubation at 37°C on an agar plate containing MHA medium supplemented with 10 µg/ml of lysozyme dimer. The MRSA strain formerly resistant to Biseptol, trimethoprim, and sulfamethoxazole regained its susceptibility to Biseptol, visible as a growth inhibition zone around the disk impregnated with Biseptol (right plate), while the control plate (left plate) with lysozyme dimer but without Biseptol did not inhibit the growth of the same strain.

### DETAILED DESCRIPTION OF THE INVENTION

The term "antibiotics" in the context of the present invention is understood to comprise any pharmaceuticals or chemotherapeutics which are known to inhibit the proliferation of microbes.

The term "microbes" as used herein relates to self-propagating prokaryotic or eukaryotic cells, and particularly to pathogenic bacteria. One of the best known and clinically most relevant bacterial species is the gram-positive strain Staphylococcus aureus which therefore has been investigated in more detail and has been made the subject of the follwing examples.

It is an object of the present invention to provide a method and a pharmaceutical compositions effective in the treatment of microbial infections in humans and animals caused by microbes resistant to one or more antibiotics or chemotherapeutics.

The composition comprises an effective amount of lysozyme dimer and can be in liquid or solid form or can be a gel or an ointment, as described for instance in WO 96/21463. It contains the active ingredient lysozyme dimer at a concentration of from 0.01 to 20 mg per ml for liquid preparations, gels or ointments, or from 0.01 to 20 mg/mg for solid preparations. Any pharmaceutically and physiologically acceptable buffer solution, such as PBS, may be used as a carrier, optionally supplemented with further additives common in the art. The dosage for in vivo applications ranges from 0.01 to 5 mg lysozyme dimer per kg body weight of the treated individual, a range of 0.02 to 0.5 mg/kg being preferred.

In order to gain a better understanding of the invention described herein, the following examples are set forth. The examples are for illustrative purposes and are not to be construed as limiting this invention in any respect.

### Example 1: Polymerase (PCR) identification of the mecA gene

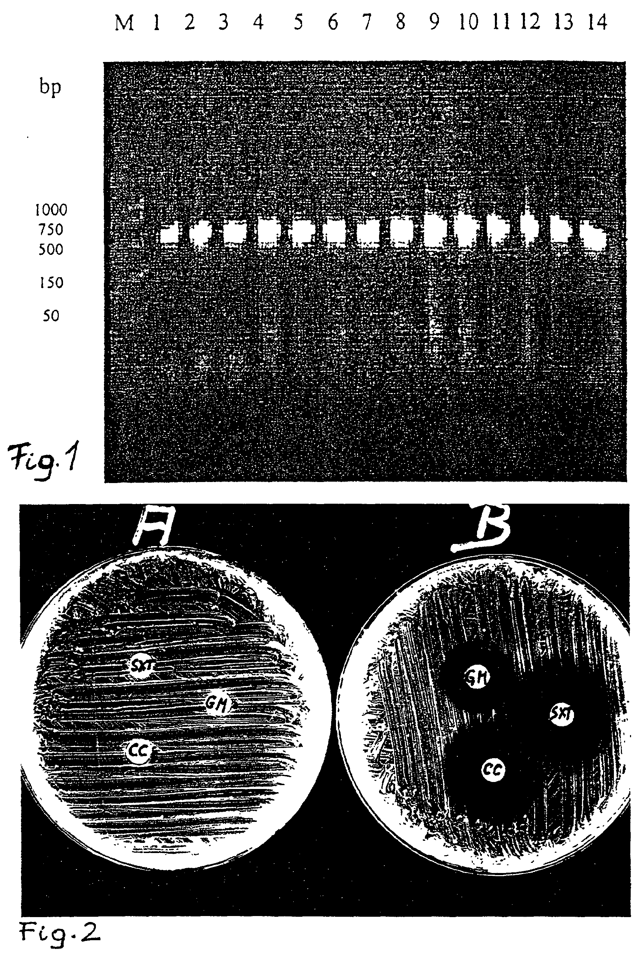

Polymerase (PCR) identification of the mecA gene through DNA duplication was done according to a known procedure (9):
The assays were conducted on a 24-hour-old MRSA culture in enriched agar medium (Biomed Wytwornia Surowic i Szczepionek) previously assayed according to the disk method. 10 to 30 cultures were suspended in 100 µl of TE buffer (10 mM Tris pH 8.0, 1mM EDTA pH 8.0). 10 µl of lysostaphin water solution (Sigma) at a concentration of 10 mg/ml was added to the suspension and incubated for 10 min at 37°C. Following incubation, 200 µl of LT buffer (which contains non-ionic detergent and chalotropic salts) and 20 µl of K-proteinase solution (20 mg/ml, A&A Biotechnology) were added. The solution was shaken for 10 sec and then incubated for 2 hours at 37°C, moved to a 75°C water bath and incubated for 5 min. Following incubation, it was vortexed for 20 sec and centrifuged for 3 min at 13,000 rpm. The supernatant was superimposed onto the columns located in Eppendorf test tubes and centrifuged for 1 min at 13,000 rpm. The columns were flushed with 500 µl of A1 solution (70% buffered alcohol solution) and centrifuged for 1 min at 13,000 rpm. The columns were placed inside new Eppendorf test tubes and DNA was eluted from the bottoms of the columns of 100 µl of elution buffer ( 10 mM Tris HCI, pH 8.5) previously heated to 75°C for 5 min. Following incubation, the sample was centrifuged for 30 sec. at 13,000 rpm, which made it possible to isolate the DNA of the strains. The volume of the solution ready for the PCR was 50 µl and its ingredients were: 5 µl of PCR, 10× buffer (1 mM Tris HCI pH 9.0,15 mM MgCl₂), 1 µl of dNTP (dATP, dCTP, dGTP and dTTP at 10 mM concentration each; Qiagen), 2 µl of each starter (0.25 µM oligonucleotides were synthesized by Prof. Markiewicz of the Bioorganic Chemistry Institute at the Polish Academy of Sciences in Poznan), 1.25 µl of Taq Polymerase (5 U/µl; Qiagen), 28.5 µl of H₂O and 10 µl of isolated DNA. As the PCR starter sequences the following oligonucleotides have been used :
SEQ ID NO.1 (5'-AAAATCGATGGTAAAGGTTGGC-3') corresponding to the 1282 to 1303 bp sequence, and
SEQ ID NO. 2 (5'-AGTTCTGCAGTACCGGATTTGC-3') corresponding to the 1794 to 1814 bp sequence in the mecA gene.
The amplification reaction was conducted in the Mastercycler 5330 thermocycler (by Eppendorf) according to the following program: 94°C 2 min; 40 cycles: 94°C 30 sec; 55°C 30 sec; 72°C 1 min. The product of amplification underwent electrophoresis in 3% agarose gel (Promega) in 1 TBE buffer in the presence of ethidene bromide and with the use of an appropriate model of DNA dimensions. The mecA gene was identified in methicillin resistant isolates through the amplification of DNA fragments measuring approximately 530 bp (Fig. 1).

### Example 2: Evaluation of the reversal of antibiotic resistance in multi-resistant strains of Staphylococcus aureus (MSSA and MRSA).

Included in the study were 26 strains of Staphylococcus aureus isolated from samples of clinical material (wound smears, pus and throat smears). Of the 26 cultured strains, 13 collected from the hospitalised patients were found to be methicillin resistant (MRSA). The other isolates were collected from patients not staying in hospitals. Lysozyme dimer was applied in liquid form, dissoleved in a physiological PBS solution (termed KLP-602; Bachem AG, Switzerland)

### Diagnostics:

The *Staphylococcus* strains were cultured according to the standard bacteriological procedure (7) and biochemically identified with API ID 32 STAPH (bioMerieux) tests. In addition, all strains identified as *Staphylococcus aureus* underwent a slide test (Cormay ST-80) for the presence of bonded coagulase (the clumping factor) and a test tube test for the generation of free coagulase.

### Assays of drug susceptibility in S.aureus strains:

The susceptibility of *S.aureus* strains to the selected antibacterial drugs (erythromycin, clindamycin, gentamycin, doxycycline, co-trimoxazole, vancomycin [hydrochloride], and teicoplanine) was assayed in the Mueller-Hinton 2 agar (MHA) medium (bioMerieux) in accordance with the Polish NCCLS recommendations (8). Resistance to methicillin was assayed according to the disk-diffusion method with the use of 1 µg and µ5 g oxacillin disks (bioMerieux) and Mueller-Hinton 2 agar with 5% NaCI (MHA with 5% NaCl, bioMerieux). Antibiograms were incubated for 24 hours at 37°C. In the *S.aureus* strains identified as methicillin resistant, the mecA gene was also identified (Fig. 1). The MR3 resistant heterogenic strain from the collection of the State Reference Center of Poland was used as the master strain.

Of the 26 clinical *Staphylococcus aureus* isolates, 13 strains were identified as MRSA according to the PCR technique (Fig. 1) and 13 strains were identified as MSSA. All MRSA strains showed resistance to MLS antibiotics (macrolides, lincosamides, and streptogramine B) and to gentamycin, doxycycline and co-trimoxazole. The MSSA strains were resistant to gentamicin, doxycycline and co-trimoxazole. Drug susceptibility was reevaluated after lysozyme dimer was added to the Mueller-Hinton medium in concentrations of 5 µg/ml, 10 µg/ml, and 20 µg/ml. The results are presented in Tables 1 and 2, and in Fig. 2.

**Table 1:**

| Results of a drug susceptibility study of MSSA and MRSA strains before and after application of lysozyme dimer (LD) at a dose of 10 µg/ml | | | | | | | | | |
|---|---|---|---|---|---|---|---|---|---|
| Strains tested | No. of strains | GM (mm) | GM+LD (mm) | CC (mm) | CC+LD (mm) | D (mm) | D+LD (mm) | SXT (mm) | SXT+L D (mm) |
| MSSA | 8 | 6 | 15 | 16 | 20 | 8 | 11 | 6 | 18-20 |
| MRSA | 10 | 6 | 15 | 6 | 20 | 6 | 6 | 6 | 15-18 |
| GM = Gentamicin (bioMerieux); CC = Clindamycin (bioMerieux); D = Doxycycline (bioMerieux); SXT = Co-trimoxazole (bioMerieux), LD= Lysozyme dimer | | | | | | | | | |

**Table 2:**

| Results of an in vitro drug susceptibility study relative to the dosage of lysozyme dimer | | | |
|---|---|---|---|
| Dose of LD (µg/ml) | GM + LD (mm) | C + LD (mm) | SXT + LD (mm) |
| 5 | 14 | 18 | 14 |
| 10 | 15 | 20 | 18 |
| 20 | 15 | 19 | 15 |
| GM = Gentamicin (bioMerieux); CC = Clindamycin (bioMerieux); D = Doxycycline (bioMerieux); SXT = Co-trimoxazole (bioMerieux), LD= Lysozyme dimer | | | |

The results show that lysozyme dimer restored full susceptibility to aminoglycosides (gentamicin) and co-trimoxazole in previously resistant MSSA and MRSA strains. MRSA strains resistant to clindamycin became moderately resistant to it following the application of lysozyme dimer. Thus, lysozyme dimer is definitely causing antibiotic resistance reversal in multiple drug resistant strains.

A control test presented in Table 3 and Figures 3A and 3B shows that lysozyme dimer alone when applied in concentrations of 10 µg/ml and 20 µg/ml does not inhibit the proliferation of MRSA and MSSA strains.

**Table 3:**

| Results of an *in vitro* study of the effect of lysozyme dimer on the growth of MSSA and MRSA strains | | | |
|---|---|---|---|
| Strains tested | Number of strains | Dose of lysozyme dimer | |
| | | 10 µg/ml | 20 µg/ml |
| MSSA | 8 | No growth inhibition | No growth inhibition |
| MRSA | 10 | No growth inhibition | No growth inhibition |

### Example 3: Evaluation of the reversal of antibiotic resistance in an MRSA strain resistant to Biseptol, trimethoprim and sulfamethoxazole.

Example 2 was repeated for a multiresistant S.aureus strain that was identified to be resistant to Biseptol, trimethoprim and sulfamethoxazole. Lysozyme dimer was added to the MHA medium at a concentration of 10 µg/ml. A Biseptol-impregnated disk was placed in the centre of the agar plate while in the control plate the disk was not impregnated with any antibiotic. After incubation of 18 hours at 37°C the experimental agar plate showed a clearly visible growth inhibition zone around the Bioseptol-impregnated disk (Fig. 4).

### References:

1. Jevons P.M.: "Celbenin" resistant staphylococci. Br. Med. J., 1961, 1, 124-125.
2. Berger-Bachi B: Expression of resistance to methicillin. Trends Microbiol 1994; 2: 389-393
3. Martin M.A.: Methicillin-resistant Staphylococcus aureus: the persistent nosocomial pathogen. Curr. Clin. Top. Infect. Dis., 1994, 14, 170-191.
4. Smith T.L., Pearson M.L., Wilcox K.R., Cruz C., Lancaster M.V. Robinson-Dunin B., Tenover F.C., Zervos M.J., Band J.D., White E., Jarvis W.R.: Emergence of vancomycin resistance in Staphylococcus aureus. N. Engl. J. Med., 1999, 340-501.
5. Voss A., Milatovic D., Wallrauch-Schwarz C., Rosdahl V.T., Braveny I.: Methicillin-resistant Staphylococcus aureus in Europe. Eur. J. Clin. Microbiol. Infect. Dis., 1994, 13, 50-55.
6. Panlilio A.L., Culver D.H., Gaynes R.P., Banerjee S., Henderson T.S., Tolson J.S., Martone W.J.: Methicillin-resistant Staphylococcus aureus in U.S. hospitals, 1975-1991. Infection Control and Hospital Epidemiology, 1992, 13, 582-586.
7. Kloos W.E., Bannerman T.L.: Staphylococcus and Micrococcus. In: Manual of clinical Microbiology (ed. P.R. Murrag, E.J. Baron, M.A. Pfaller, F.C. Tenover, R.H. Yolken). Am Soc. Microbiol., Washington, 1995, 26.
8. National Comitee for Clinical Laboratory Standards: Performance Standards for Antimicrobial Disk Susceptibility tests - Third edition; Approved Standard. NCCLS document 1997, M2-A6.
9. Murakami K., Mimamide W., Wada K., Nakamura E., Teraoka H., Watanabe S.: Identification of methicillin-resistant strains of staphylococci by polymerase chain reaction. J. Clin. Microbiol., 1991, 29, 2240-2244.

## Claims

1. Use of lysozyme dimer for rendering antibiotic-resistant microbes antibiotic-sensitive.

2. Use of lysozyme dimer as an active ingredient in the manufacture of a pharmaceutical composition for adminstration to a recipient suffering from an infection by antibiotic-resistant microbes, for restoring the recipient's susceptibility towards antibiotic treatment.

3. Use of lysozyme dimer according to claim 2, wherein the microbes are resistant to one or more antibiotics and wherein susceptibility toward at least one antibiotic is restored.

4. Use of lysozyme dimer according to claim 2 or 3, wherein the composition is for administration before, simultaneously with, or after treatment with an antibiotic.

5. Use of lysozyme dimer according to any one of claims 2 to 4, wherein the microbial infection is by gram-positive bacteria.

6. Use of lysozyme dimer according to claim 5, wherein the microbial infection is by staphylococci, in particular *Staphylococcus aureus.*

7. Use of lysozyme dimer according to any one of the preceding claims, wherein the microbes are resistant to at least one antibiotic selected from the group consisting of macrolides, lincosamides, glycoside antibiotics and streptogramine B.

8. Use of lysozyme dimer according to claim 7, wherein said antibiotic is selected from the group consisting of gentamicin, clindamycin, doxycyline and co-trimoxacol.

9. Use of lysozyme dimer according to any one of claims 2 to 8, wherein the composition is suitable for administration of a single or repeated dose of 0.01 to 5 mg lysozyme dimer per kg body weight of a recipient.


SEQUENCE LISTING

<110> Nika Health Products Limited

<120> Reversal Of Antibiotic Resistance

<130> BLP-4797-EU (LD Anti-Resistance)

<140>
<141>

<160> 2

<170> PatentIn Ver. 2.1

<210> 1
<211> 22
<212> DNA
<213> Artificial Sequence

<220>
<223> Description of Artificial Sequence: sequence
      corresponding to base pairs 1282 to 1303 of mecA
      gene of Staphylococcus aureus

<400> 1
aaaatcgatg gtaaaggttg gc                                          22

<210> 2
<211> 22
<212> DNA
<213> Artificial Sequence

<220>
<223> Description of Artificial Sequence: sequence
      corresponding to base pairs 1794 to 1814  of mecA
      gene of Staphylococcus aureus

<400> 2
agttctgcag taccggattt gc                                          22